(19) Europäisches Patentamt
European Patent Office
Office européen des brevets

(11) **EP 2 929 906 A1**

(12) **EUROPÄISCHE PATENTANMELDUNG**

(43) Veröffentlichungstag:
**14.10.2015 Patentblatt 2015/42**

(51) Int Cl.:
***A61M 16/00*** *(2006.01)* ***A61B 5/08*** *(2006.01)*

(21) Anmeldenummer: **15162130.7**

(22) Anmeldetag: **01.04.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **11.04.2014 AT 502742014**

(71) Anmelder: **EMS Handels Gesellschaft m.b.H. 2100 Korneuburg (AT)**

(72) Erfinder: **Ortner, Alfred 2111 Mollmannsdorf (AT)**

(74) Vertreter: **Müllner, Martin et al Weihburggasse 9 1014 Wien (AT)**

(54) **MESS- UND STEUERVORRICHTUNG FÜR EIN BEATMUNGSGERÄT**

(57) Die Erfindung betrifft eine Mess- und Steuervorrichtung für ein Beatmungsgerät, insbesondere zur Steuerung der assistierten Beatmung, umfassend einen Sensorabschnitt zur Erfassung der Lungenfunktion, eine Auswerteeinheit, welche die vom Sensorabschnitt gelieferten Signale verarbeitet und gegebenenfalls über eine Ein- Ausgabevorrichtung verfügt, sowie eine Steuereinheit, welche aufgrund der von der Auswerteeinheit ermittelten Daten Steuerimpulse an ein Beatmungsgerät übermittelt. Der Sensorabschnitt umfasst mindestens in zwei transversalen Ebenen (10,12) des Bereichs der Lunge auf der Haut angeordnete Elektrodenpaare (6,7,8,9) für die Messung der Impedanz. Die Auswerteeinheit verarbeitet die Signale im Rahmen einer kontinuierlichen sektionalen Kreuz-Impedanzmessung und berechnet den jeweils aktuellen Ventilationsgrad der Lunge bzw. eines Lungenabschnitts und übermittelt über die Steuereinheit entsprechende Steuerimpulse, insbesondere über den Zeitpunkt eines Beatmungshubs, an ein Beatmungsgerät.

Fig. 3

EP 2 929 906 A1

**Beschreibung**

**Technisches Gebiet**

Die Erfindung betrifft eine Mess- und Steuervorrichtung für ein Beatmungsgerät, insbesondere zur Steuerung der assistierten Beatmung, umfassend einen Sensorabschnitt zur Erfassung der Lungenfunktion, eine Auswerteeinheit, welche die vom Sensorabschnitt gelieferten Signale verarbeitet und gegebenenfalls über eine Ein- Ausgabevorrichtung verfügt, sowie eine Steuereinheit, welche aufgrund der von der Auswerteeinheit ermittelten Daten Steuerimpulse an ein Beatmungsgerät übermittelt.

**Stand der Technik**

Die künstliche Beatmung, insbesondere die assistierte Beatmung von Patienten, stellt große Herausforderungen an die Mess- und Regeltechnik, da eine zu geringe Beatmung einen Sauerstoffmangel verursacht, während eine Beatmung mit zu großen Volumen oder zum falschen Zeitpunkt zu Verletzungen in der Lunge führen kann oder mit anderen Komplikationen für den Patienten verbunden ist.

Als besonders kritisch hat sich die Beatmung von zu früh geborenen Kindern herausgestellt, da hier nur geringe Beatmungsvolumen von oft nur 10 cm$^3$ Luft für einen Atemzug ausreichen. Gerade bei dieser Patientengruppe mit einem unreifen Atmungssystem und noch nicht richtig ventilierenden Lungen soll eine Beatmung den natürlichen Atemvorgang unterstützen, aber keinesfalls soll die Spontanatmung durch die maschinelle Beatmung gestört werden. Es müssen daher die Signale des Körpers sehr sensibel und über lange Zeit, eventuell über mehrere Monate, kontinuierlich abgenommen und ausgewertet werden.

Im Stand der Technik bekannte nicht invasive Verfahren, wie beispielsweise die Impedanztomographie, arbeiten verfahrensbedingt mit relativ hohen Strömen von mehreren Milliampere, die durch den Körper fließen, was bei Frühgeborenen aufgrund der hoch sensiblen Gewebe, insbesondere für ein Langzeitmonitoring, nicht akzeptabel ist.

Die DE 10301202 beschreibt beispielsweise ein derartiges Beatmungssystem mit hohen Strömen basierend auf der Impedanztomographie eines Lungenquerschnitts sowie einem bidirektionalen Datenaustausch zwischen dem Beatmungsgerät und der Sensorik. Ein weiterer großer Nachteil der bekannten Impedanztomographie ist die hohe Anzahl der benötigten Sensorelektroden, welche für die Erfassung eines einzelnen Querschnitts benötigt werden. Um ein brauchbares Ergebnis zu bekommen, werden in der Praxis 16 oder gar 32 Messelektroden benötigt, welche innerhalb einer einzigen Transversalebene platziert werden müssen. Dies bedeutet einen sehr hohen Aufwand für das Personal, welches die Elektroden platzieren muss und behindert weitere Pflegemaßnahmen erheblich. Bei Neugeborenen ist das korrekte Platzieren derart vieler Elektroden aufgrund des mangelnden Platzes am Brustkorb praktisch gar nicht möglich. Die Berechnungsalgorithmen für die Impedanztomographie sind weiters äußerst anspruchsvoll, was die verfügbare Rechenleistung der Auswerteeinheiten betrifft, weshalb eine Echtzeitüberwachung ebenfalls schwierig ist. Aufgrund des Berechnungsvorgangs bei dieser Art der Messung genügt auch schon der Ausfall einer einzigen Elektrode, um das Ergebnis unbrauchbar zu machen.

Aus all diesen genannten Gründen sind derartige Verfahren auch nicht im Zusammenhang mit dem Auslösen der assistierten Beatmung beschrieben, sondern lediglich als kurzzeitige Monitoringvarianten, um Lungenvolumen und Ventilationsgrad zu bestimmen. Die gewonnenen Daten können somit nur zur Einstellung von Beatmungsvolumina verwendet werden, oder zur Kontrolle der richtigen Lage eines Tubus.

Eine weitere bekannte Methode zur Steuerung der assistierten Beatmung ist die Messung des Luftflusses am Mund bzw. der Nase, wodurch der Beginn eines Atemzugs detektiert werden kann, woraufhin ein Beatmungshub ausgelöst wird. Diese Methode kann jedoch nur beim Erwachsenen eingesetzt werden, da bei Frühgeborenen die entsprechende Sensorik bereits einen Luftfluss von 0,1 cm$^3$ bis maximal 0,5 cm$^3$ sicher detektieren müsste. Allein aufgrund der Artefaktbildung durch unwillkürliche Bewegungen des Patienten ist eine sichere Detektion derart kleiner Volumen nicht möglich.

**Kurzbeschreibung der Erfindung**

Es ist somit Aufgabe der vorliegenden Erfindung eine Mess- und Steuervorrichtung zu schaffen, welche die oben genannten Nachteile beseitigt und es auch bei Frühgeborenen erlaubt, ein kontinuierliches nichtinvasives Monitoring der Lungenfunktion durchzuführen und ein Beatmungsgerät auch für die assistierte Beatmung sicher anzusteuern. Weiters soll die Vorrichtung einfach in der Handhabung durch das Pflegepersonal sein, eine hohe Ausfallsicherheit auch beim Ausfall einzelner Elektroden aufweisen und möglichst wenig Platz einnehmen, sodass andere Pflegemaßnahmen nicht behindert werden.

Diese Aufgabe wird erfindungsgemäß durch eine Mess- und Steuervorrichtung gelöst, die dadurch gekennzeichnet ist, dass der Sensorabschnitt mindestens in zwei transversalen Ebenen des Bereichs der Lunge auf der Haut angeordnete Elektrodenpaare für die Messung der Impedanz umfasst, und dass die Auswerteeinheit die Signale im Rahmen einer kontinuierlichen sektionalen Kreuz-Impedanzmessung verarbeitet und den jeweils aktuellen Ventilationsgrad der Lunge bzw. eines Lungenabschnitts berechnet und über die Steuereinheit entsprechende Steuerimpulse, insbesondere über den Zeitpunkt eines Beatmungshubs, an ein Beatmungsgerät übermittelt.

Durch die sektionale Kreuz-Impedanzmessung wird nicht wie bei der Impedanztomographie ein genaues Abbild eines einzelnen Lungenquerschnitts erzeugt, sondern vielmehr werden für die einzelnen wenigen Segmente, welche über die gesamte Lungenfläche verteilt sind Impedanzwerte ausgewertet. Es hat sich nun in überraschender Weise herausgestellt, dass diese wenigen Messwerte ein sehr genaues Bild über den Ventilationsgrad der Lunge insgesamt sowie auch für die einzelnen Segmente liefern. Je nach Anzahl der Segmente, wobei für jedes Segment ein Messelektrodenpaar vorgesehen ist, können neben dem Ventilationsgrad auch Flüssigkeitsansammlungen oder Schädigungen von Lungenbereichen genau detektiert werden.

Es ist ein weiteres Merkmal der Erfindung, dass für die Einspeisung des für die Impedanzmessung nötigen sinusförmigen Konstantstroms drei Einspeiseelektroden auf der Haut in einer Transversalebene im Bereich der Lunge angeordnet sind, wobei bevorzugt eine Elektrode dorsal und zwei Elektroden lateral angeordnet sind.

Durch diese Anordnung der Einspeiseelektroden bekommt man eine besonders günstige räumliche Auflösung der gemessenen Impedanzen, insbesondere auch in lateraler Richtung.

Gemäß einem weiteren Merkmal der Erfindung ist es vorgesehen, dass Messelektrodenpaare in drei regelmäßig voneinander beabstandeten Transversalebenen im Bereich der Lunge auf der Haut angeordnet sind.

Bei Messungen in drei Ebenen wird die Auflösung weiter verbessert.

Dabei ist es gemäß einem weiteren Merkmal der Erfindung vorgesehen, dass der Sensorabschnitt in jeder Transversalebene jeweils zwei Elektrodenpaare umfasst. Es werden dabei je Sektor eine Elektrode am Rücken und eine gegenüber auf der Brust platziert. Bei zwei Transversalebenen werden also 4 Segmente mit insgesamt 8 Messelektroden überwacht. Bei drei Transversalebenen 6 Segmente mit insgesamt 12 Messelektroden. Da nur 4 bzw. 6 Messelektroden über den gesamten Brustkorb verteilt auf der Vorderseite angebracht sind, ist selbst bei einem Frühgeborenen ausreichend Fläche für das Platzieren aller Elektroden vorhanden. Außerdem bleibt insbesondere im Brustbereich genügend Platz für andere pflegerische Maßnahmen. Die Einspeiseelektroden und die verbleibenden Messelektroden sind seitlich oder am Rücken angebracht und stellen somit ebenfalls nur eine minimale Behinderung dar.

Ferner ist es ein Merkmal der vorliegenden Erfindung, dass die Messfrequenz in einem Messbereich von weniger als 10 KHz bevorzugt von 5-10 KHz liegt. Die eingespeiste Stromstärke soll gemäß einem weiteren Merkmal der Erfindung einen Wert von 500 µA nicht übersteigen. Die geringe Stromstärke ermöglicht es, ohne Schädigungen für das Gewebe auch bei empfindlichen Patienten wie Frühgeborenen ein Langzeitmonitoring durchzuführen, und dadurch den Entwicklungsgrad der Lunge und damit die Ventilationskapazität zu jedem Zeitpunkt zu überwachen. Da solche Entwicklungsschritte der Lunge oftmals binnen Stunden stattfinden und das Lungenvolumen dabei drastisch zunimmt, kann über den gesamten Zeitraum hinweg eine optimale Luftversorgung sichergestellt werden. Durch die Wahl einer entsprechend hohen Messfrequenz kann sichergestellt werden, dass bei der Auswertung des Signals die Frequenz klar von den anderen Störsignalen, wie beispielsweise des Herzschlages, isoliert werden kann.

Gemäß einem weiteren Merkmal der Erfindung ist es vorgesehen, dass die Steuereinheit einen Steuerimpuls übermittelt, sobald von der Auswerteeinheit eine Luftvolumenszunahme in der Lunge von max. 0,5 cm$^3$, bevorzugt von max. 0,1 cm$^3$, zu Beginn einer Einatemphase ermittelt wurde. Bei der assistierten Beatmung wird somit bereits ein sehr geringer Anstieg des Lungenvolumens durch die Spontanatmung detektiert. Ein erschwerender Faktor der assistierten Beatmung ist es, dass die Spontanatmung keinem durchgehend regelmäßigen Muster folgt, sondern dass es auch zu längeren, nicht vorhersehbaren Pausen kommt. Da durch die genaue Messmethode, welche mittels einer Vorrichtung gemäß der Erfindung durchgeführt wird, bereits sehr früh der Beginn eines Einatemvorgangs detektiert werden kann, kann dieses Signal verwendet werden, um einen einfachen Steuerbefehl an ein entsprechendes Beatmungsgerät zu übermitteln. Dabei hat die Vorrichtung gemäß der Erfindung den Vorteil, dass sie mit so gut wie jedem Beatmungsgerät kompatibel ist, was die Flexibilität in der Anwendung erhöht. Es wird keine bidirektionale Kommunikation zwischen Beatmungsgerät und Vorrichtung der Erfindung benötigt, vielmehr ermittelt die Vorrichtung autonom alle notwendigen Daten und sendet bei Bedarf ein entsprechendes Triggersignal.

**Kurze Beschreibung der Zeichnungsfiguren**

Die Erfindung wird nun näher anhand der beiliegenden Figuren beschrieben, wobei

Fig. 1 einen schematischen Querschnitt durch eine Transversalebene eines menschlichen Thorax mit angeordneten Einspeiseelektroden zeigt,
Fig. 2 einen schematischen Querschnitt durch eine Transversalebene eines menschlichen Thorax mit angeordneten Messelektroden zeigt,
Fig. 3 eine schematische Durchsicht durch einen menschlichen Thorax von vorne zeigt mit in drei Transversalebenen angeordneten Elektroden,
Fig. 4 eine schematische Durchsicht durch einen menschlichen Thorax von vorne zeigt mit einer groben Einteilung der vermessenen Segmente,
Fig. 5 ein Diagramm , welches die über einen Zeitabschnitt gewonnen Messdaten zeigt und
Fig. 6 einen Detailausschnitt der gewonnen Atemvolumenskurve mit einer Darstellung der Berechnung des Auslösepunkts für ein Steuersignal.

## Beschreibung der Ausführungsarten

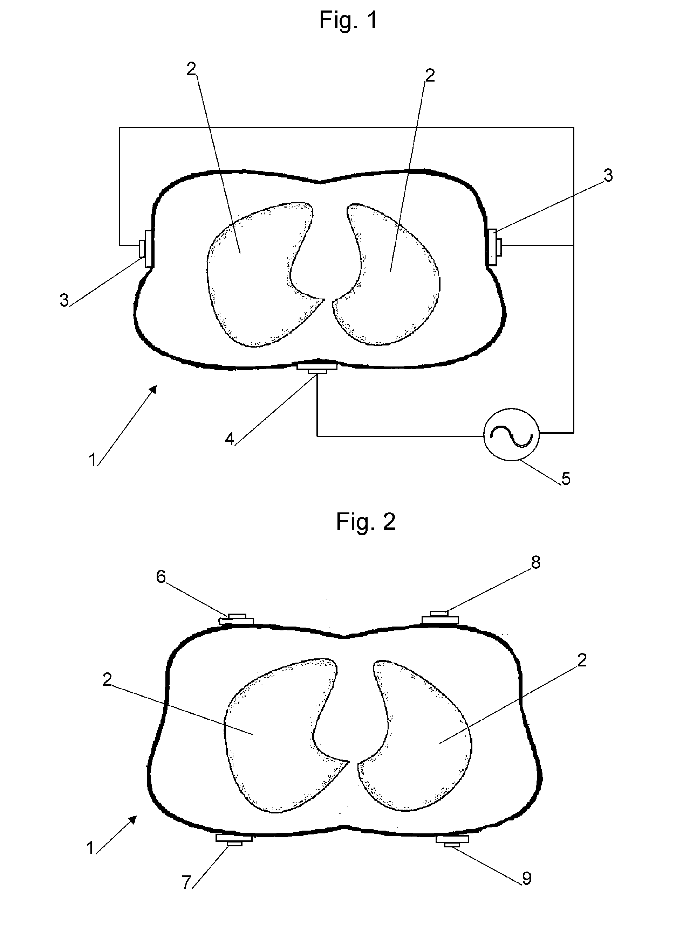

Der in der Fig. 1 im Querschnitt dargestellte menschliche Thorax 1 befindet sich in Rückenlage. Bis auf die Lungenflügel 2 sind alle anderen Organe zwecks Übersichtlichkeit nicht eingezeichnet. Weiters sind die drei Einspeiseelektroden 3,4 auf der Haut angeordnet, wobei eine der Einspeiseelektroden 4 am Rücken angeordnet ist und zwei der Einspeiseelektroden 3 an den Seiten. Alle Einspeiseelektroden sind mit einer Wechselstromquelle 5 verbunden, wobei ein sehr schwacher Strom von wenigen mA ausreicht, um eine zuverlässige Messung durchzuführen.

In Fig. 2 ist eine Querschnittsdarstellung des Thorax 1 dargestellt, bei der 4 Messelektroden 6,7,8,9 in einer Transversalebene angeordnet sind. Dabei bilden jeweils die beiden linken Messelektroden 6,7 und rechten Messelektroden 8,9 ein Elektrodenpaar.

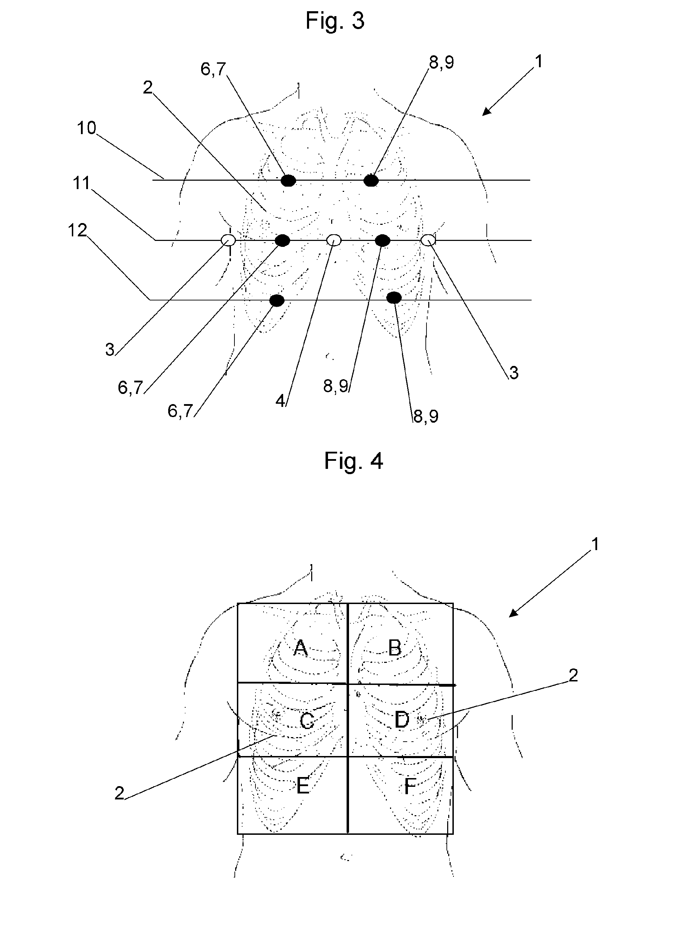

Fig. 3 zeigt eine Aufsicht auf einen Thorax 1, mit Messelektrodenpaaren 6,7,8,9 in drei Transversalebenen 10,11,12. Da die am Rücken befindlichen Messelektroden 7,9 genau unterhalb der brustseitigen Messelektroden 6,8 angeordnet sind, sind diese in der Zeichnung nicht separat ersichtlich. Die Fig. 4 zeigt schematisch die durch die Messelektrodenanordnung gemäß Fig. 3 abgedeckten Messsektoren A,B,C,D,E und F. Die Sektoren A und B werden dabei von den Messelektrodenpaaren 6,7,8,9 in der oberen Transversalebene 10 erfasst, die Sektoren C und D von den Messelektrodenpaaren 6,7,8,9 in der mittleren Transversalebene 11 und die Sektoren E und F von den Messelektrodenpaaren 6,7,8,9 in der unteren Transversalebene 12. Bei dieser Anordnung befinden sich die Einspeiseelektroden 3,4 ebenfalls in der mittleren Transversalebene 11. Bei einer Anordnung mit nur vier überwachten Sektoren in zwei Transversalebenen, würden in der mittleren Transversalebene 11 lediglich die Einspeiseelektroden 3,4 angeordnet sein, jedoch keine Messelektrodenpaare 6,7,8,9.

Die Differenzmessungen zwischen den jeweils übereinanderliegenden linken 6,7 und rechten 8,9 Messelektrodenpaaren liefern Aussagen über die relative Änderung der Impedanz und damit über die Ventilation der Lunge im jeweiligen Abschnitt. Die Differenzmessungen zwischen den jeweils nebeneinander liegenden vorderen 6,8 und hinteren 7,9 Messelektroden liefern Rückschlüsse über Flüssigkeitsverlagerungen bei liegender Lage eines Patienten.

Die gemessenen Änderungen der Impedanz in einem Segment während eines Atemzugs entsprechen der Änderung des Gasvolumens im entsprechenden Lungenabschnitt. Wenn man davon ausgeht, dass das vom Beatmungsgerät zugeführte Luftvolumen nur in die vorhandenen Lungensegmente verteilt werden kann, so lässt sich die örtliche Verteilung des Gesamtvolumens wie folgt berechnen:

$$V_A = \frac{\Delta Z_A}{\sum \Delta Z} * V_{in}$$

wobei $V_A$ dem Gasvolumen im Segment A entspricht,

$\Delta Z_A$ der Impedanzänderung im Segment A entspricht,

$\Sigma \Delta Z$ der Summe der Impedanzänderungen in allen Segmenten entspricht und

$V_{in}$ dem Gasvolumen entspricht, welches vom Beatmungsgerät geliefert wird.

Die Summe der gemessenen Gasvolumina entspricht dem zugeführten Gasvolumen und ergibt 100%. Daraus lässt sich der prozentuelle Anteil in den einzelnen Segmenten errechnen.

Da außer in Ausnahmefällen im Allgemeinen die gesamte Lunge relativ gleichmäßig ventiliert ist, ist die Vorrichtung auch relativ ausfallsicher, da selbst bei Ausfall eines einzelnen Segments, beispielsweise durch Abfallen einer Messelektrode, immer noch ausreichend aussagekräftige Daten gewonnen werden können.

Die ermittelten Daten können auf der Ein- Ausgabevorrichtung zur Verfügung gestellt werden und liefern dem Arzt Daten, die bei der Diagnose des Lungenzustands und der Einstellung eines Beatmungsgeräts hilfreich sind. Gleichzeitig kann die kontinuierliche Messung zur direkten Ansteuerung eines Beatmungsgeräts für die assistierte Beatmung eingesetzt werden.

Über die Ein- Ausgabevorrichtung können nicht nur das zur Verfügung gestellte Gasvolumen vom Beatmungsgerät eingegeben werden, sondern auch weitere Faktoren, wie beispielsweise prozentuelle Schädigungsfaktoren einzelner Lungensegmente, die vom Arzt zuvor diagnostiziert wurden. Ausgehend von diesem herabgesetzten Startwert für einzelne Segmente können dann Verbesserungen oder Verschlechterungen des Zustands auch auf das einzelne Segment genau bestimmt werden.

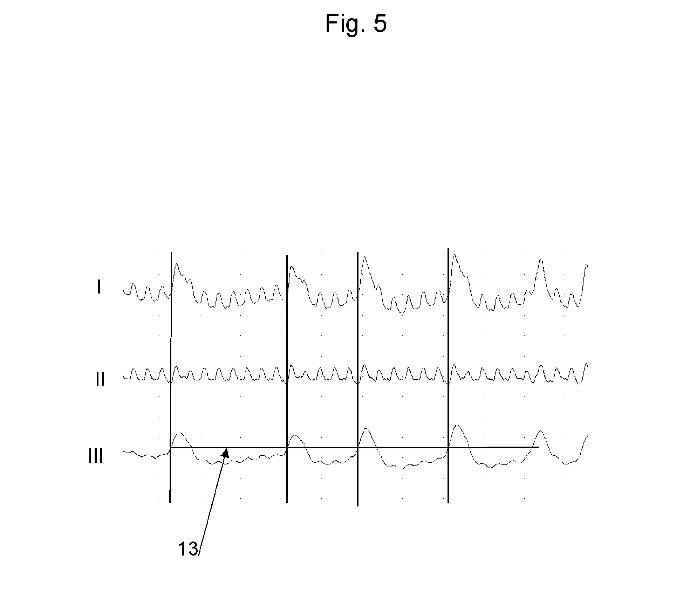

Die in Fig. 5 gezeigten beispielhaften Messdaten zeigt in der Kurve I das gewonnene Volumensignal über einen Zeitabschnitt, wie es aus den Differenzmessungen der Impedanzen von einer Anordnung mit 4 Sektoren gewonnen wurde. Die Auswerteeinheit sampelt das von den Differenzverstärkern gelieferte Signal mit einem Analog-Digital-Wandler mit bis zu 100 KHz. Zur Minimalisierung von Störsignalen und Bewegungsartefakten werden diese Daten in Abständen von minimal 1 ms einer Fast-Fourier-Transformation (FFT), beispielsweise mittels Goertzel-Algorithmus, unterzogen. Der Spitzenwert bei der Frequenz der Konstantstromquelle ergibt einen Anzeigepunkt des Ausgangssignals. Somit ergeben sich bei einer Abtastzeit von 1 ms 1000 Messpunkte pro Sekunde. Das Signal wird mittels Filtern weiterverarbeitet.

Das ursprünglich gewonnene Messsignal setzt sich aus der Änderung des Atemvolumens und der Volumsänderung des Herzens zusammen. Wie oben beschrieben wird mittels FFT die Frequenz des Herzsignals berechnet. Diese Frequenz bestimmt die Eckfrequenz eines nachfolgenden Hochpass Filters. Das Ausgangssignal des Filters beinhaltet nun nur das Volumssignal des Herzens (Kurve II). Durch Subtraktion dieses Signals vom ursprünglichen Messignal erhält man ein entstörtes Atemvolumssignal (Kurve III) das einer weiteren Verarbeitung, beispielsweise für eine Differenzdruckmessung oder die Berechnung der Triggerungssignale, zugeführt werden kann.

Zur Berechnung des Triggerzeitpunkts für die Übermittlung eines Signals an ein Beatmungsgerät wird auf der Ausgabevorrichtung eine horizontale Triggerlinie 13 eingeblendet, die vom Benutzer einstellbar ist. Jedesmal wenn das Signal der Kurve III die Triggerlinie von unten nach oben hin durchläuft, wird von der Vorrichtung ein entsprechendes Signal zum Beatmungsgerät gesendet (dargestellt durch senkrechte Striche). Alternativ kann der Triggerzeitpunkt auch durch automatisierte Verfahren im Gerät aufgrund von voreingestellten Grenzwerten berechnet werden.

## Patentansprüche

1. Mess- und Steuervorrichtung für ein Beatmungsgerät, insbesondere zur Steuerung der assistierten Beatmung, umfassend einen Sensorabschnitt zur Erfassung der Lungenfunktion, eine Auswerteeinheit, welche die vom Sensorabschnitt gelieferten Signale verarbeitet und gegebenenfalls über eine Ein- Ausgabevorrichtung verfügt, sowie eine Steuereinheit, welche aufgrund der von der Auswerteeinheit ermittelten Daten Steuerimpulse an ein Beatmungsgerät übermittelt, **dadurch gekennzeichnet, dass** der Sensorabschnitt mindestens in zwei transversalen Ebenen (10,12) des Bereichs der Lunge auf der Haut angeordnete Elektrodenpaare (6,7,8,9) für die Messung der Impedanz umfasst, und dass die Auswerteeinheit die Signale im Rahmen einer kontinuierlichen sektionalen Kreuz-Impedanzmessung verarbeitet und den jeweils aktuellen Ventilationsgrad der Lunge bzw. eines Lungenabschnitts berechnet und über die Steuereinheit entsprechende Steuerimpulse, insbesondere über den Zeitpunkt eines Beatmungshubs, an ein Beatmungsgerät übermittelt.

2. Mess- und Steuervorrichtung für ein Beatmungsgerät nach Anspruch 1, **dadurch gekennzeichnet, dass** für die Einspeisung des für die Impedanzmessung nötigen sinusförmigen Konstantstroms drei Einspeiseelektroden (3,4) auf der Haut in einer Transversalebene (11) im Bereich der Lunge angeordnet sind, wobei bevorzugt eine Elektrode (4) dorsal und zwei Elektroden (3) lateral angeordnet sind.

3. Mess- und Steuervorrichtung für ein Beatmungsgerät nach Anspruch 1 oder 2, **dadurch gekennzeichnet, dass** Messelektrodenpaare (6,7,8,9) in drei regelmäßig voneinander beabstandeten Transversalebenen (10,11,12) im Bereich der Lunge auf der Haut angeordnet sind.

4. Mess- und Steuervorrichtung für ein Beatmungsgerät nach einem der Ansprüche 1 bis 3, **dadurch gekennzeichnet, dass** der Sensorabschnitt in jeder Transversalebene (10,11,12) jeweils zwei Elektrodenpaare (6,7,8,9) umfasst.

5. Mess- und Steuervorrichtung für ein Beatmungsgerät nach einem der Ansprüche 1 bis 4, **dadurch gekennzeichnet, dass** die Messfrequenz bei weniger als 10 KHz, bevorzugt bei 5-10 KHz, liegt.

6. Mess- und Steuervorrichtung für ein Beatmungsgerät nach einem der Ansprüche 1 bis 5, **dadurch gekennzeichnet, dass** die über die Einspeiseelektroden (3,4) eingespeiste Stromstärke maximal 500 μA beträgt.

7. Mess- und Steuervorrichtung für ein Beatmungsgerät nach einem der Ansprüche 1 bis 6, **dadurch gekennzeichnet, dass** die Steuereinheit einen Steuerimpuls übermittelt, sobald von der Auswerteeinheit eine Luftvolumenszunahme in der Lunge von max. 0,5 cm$^3$, bevorzugt von max. 0,1 cm$^3$, zu Beginn einer Einatemphase ermittelt wurde.

## Fig. 1

## Fig. 2

Fig. 3

Fig. 4

Fig. 5

**Europäisches Patentamt**
**European Patent Office**
**Office européen des brevets**

## EUROPÄISCHER RECHERCHENBERICHT

Nummer der Anmeldung

EP 15 16 2130

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2012/041279 A1 (FREEMAN JENNY E [US] ET AL) 16. Februar 2012 (2012-02-16)<br>* Absatz [0003] *<br>* Absatz [0021] - Absatz [0030] *<br>* Absatz [0032] - Absatz [0041] *<br>* Absatz [0043] - Absatz [0052] *<br>* Absatz [0054] - Absatz [0059] *<br>* Absatz [0091] - Absatz [0140] *<br>* Absatz [0181] - Absatz [0191] *<br>* Absatz [0195] - Absatz [0200] *<br>* Absatz [0212] - Absatz [0214] *<br>* Abbildungen 23, 25, 28-32 *<br>----- | 1-7<br><br>1-7 | INV.<br>A61M16/00<br>A61B5/08 |
| Y | US 2005/065567 A1 (LEE KENT [US] ET AL) 24. März 2005 (2005-03-24)<br>* Absatz [0002] - Absatz [0015] *<br>* Absatz [0027] - Absatz [0091] *<br>* Absatz [0122] - Absatz [0124] *<br>* Abbildungen 1A-3, 7 *<br>----- | 1-7 | |
| Y | EP 0 324 275 A1 (VISVESHWARA M D INC N [US]) 19. Juli 1989 (1989-07-19)<br>* Spalte 1, Zeile 3 - Spalte 3, Zeile 23 *<br>* Spalte 3, Zeile 45 - Spalte 5, Zeile 34 *<br>* Abbildungen 1, 4 *<br>----- | 1-7 | RECHERCHIERTE SACHGEBIETE (IPC)<br><br>A61M<br>A61B |
| Y | DE 699 26 394 T2 (BOEHM STEPHAN [DE]; AMATO MARCELO B P [BR]) 1. Juni 2006 (2006-06-01)<br>* Absatz [0001] - Absatz [0029] *<br>* Absatz [0053] - Absatz [0069] *<br>* Absatz [0082] - Absatz [0086] *<br>* Abbildung 11 *<br>-----<br>-/-- | 1-7 | |

Der vorliegende Recherchenbericht wurde für alle Patentansprüche erstellt

| Recherchenort | Abschlußdatum der Recherche | Prüfer |
|---|---|---|
| München | 5. August 2015 | Aguado, Miguel |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

EP 2 929 906 A1

Europäisches
Patentamt
European
Patent Office
Office européen
des brevets

EUROPÄISCHER RECHERCHENBERICHT

Nummer der Anmeldung

EP 15 16 2130

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | FR 2 020 493 A5 (ANVAR) 10. Juli 1970 (1970-07-10) * Seite 1, Zeile 1 - Zeile 40 * * Seite 2, Zeile 13 - Seite 3, Zeile 35 * * Seite 5, Zeile 17 - Zeile 22 * * Abbildungen 1, 2 * | 1-7 | |
| Y | JAKOBSON S ET AL: "IMPEDANCE PNEUMOGRAPHY FOR DETECTION OF DISPLACEMENTS OF THE DIAPHRAGM", UPSALA JOURNAL OF MEDICAL SCIENCES, UPSALA MEDICAL SOCIETY, SE, Bd. 82, Nr. 4, 1. Januar 1977 (1977-01-01) , Seiten 241-246, XP008135393, ISSN: 0300-9734 * "Abstract", "Introduction", "Material and Methods" * * Abbildung 1 * | 1-7 | |
| Y | US 4 269 195 A (ITOH AYAO) 26. Mai 1981 (1981-05-26) * Spalte 1, Zeile 6 - Spalte 2, Zeile 4 * * Spalte 2, Zeile 20 - Spalte 4, Zeile 39 * * Abbildung 1 * | 1-7 | RECHERCHIERTE SACHGEBIETE (IPC) |
| Y | US 4 649 932 A (SMITH DENIS N [GB]) 17. März 1987 (1987-03-17) * Spalte 1, Zeile 8 - Spalte 3, Zeile 56 * * Spalte 4, Zeile 23 - Spalte 9, Zeile 14 * * Spalte 11, Zeile 65 - Spalte 12, Zeile 49 * * Abbildungen 1, 3, 5, 6, 12, 13 * | 1-7 | |
| A | WO 99/65393 A1 (MALLINCKRODT INC [US]; LAMBERT SCOTT A [US]) 23. Dezember 1999 (1999-12-23) * das ganze Dokument * | 1-7 | |

Der vorliegende Recherchenbericht wurde für alle Patentansprüche erstellt

| Recherchenort | Abschlußdatum der Recherche | Prüfer |
|---|---|---|
| München | 5. August 2015 | Aguado, Miguel |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 2130

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-08-2015

| Im Recherchenbericht angeführtes Patentdokument | Datum der Veröffentlichung | Mitglied(er) der Patentfamilie | | Datum der Veröffentlichung |
|---|---|---|---|---|
| US 2012041279 A1 | 16-02-2012 | AU | 2011289159 A1 | 14-02-2013 |
| | | CA | 2805124 A1 | 16-02-2012 |
| | | CL | 2013000205 A1 | 23-08-2013 |
| | | CN | 103153184 A | 12-06-2013 |
| | | EP | 2603138 A1 | 19-06-2013 |
| | | JP | 2014502854 A | 06-02-2014 |
| | | KR | 20130108273 A | 02-10-2013 |
| | | US | 2012041279 A1 | 16-02-2012 |
| | | WO | 2012021900 A1 | 16-02-2012 |
| US 2005065567 A1 | 24-03-2005 | US | 2005065567 A1 | 24-03-2005 |
| | | US | 2010174154 A1 | 08-07-2010 |
| EP 0324275 A1 | 19-07-1989 | DE | 3879455 D1 | 22-04-1993 |
| | | EP | 0324275 A1 | 19-07-1989 |
| | | US | 4915103 A | 10-04-1990 |
| DE 69926394 T2 | 01-06-2006 | AT | 300234 T | 15-08-2005 |
| | | AT | 457680 T | 15-03-2010 |
| | | AU | 747778 B2 | 23-05-2002 |
| | | BR | 9916092 A | 04-09-2001 |
| | | CA | 2354395 A1 | 15-06-2000 |
| | | DE | 19857090 A1 | 29-06-2000 |
| | | DE | 69926394 D1 | 01-09-2005 |
| | | DE | 69926394 T2 | 01-06-2006 |
| | | EP | 1137365 A1 | 04-10-2001 |
| | | EP | 1593341 A1 | 09-11-2005 |
| | | ES | 2341719 T3 | 25-06-2010 |
| | | JP | 4855579 B2 | 18-01-2012 |
| | | JP | 2002531207 A | 24-09-2002 |
| | | US | 2002193700 A1 | 19-12-2002 |
| | | US | 2004073130 A1 | 15-04-2004 |
| | | WO | 0033733 A1 | 15-06-2000 |
| FR 2020493 A5 | 10-07-1970 | KEINE | | |
| US 4269195 A | 26-05-1981 | JP | S5621419 B2 | 19-05-1981 |
| | | JP | S54156390 A | 10-12-1979 |
| | | US | 4269195 A | 26-05-1981 |
| US 4649932 A | 17-03-1987 | KEINE | | |
| WO 9965393 A1 | 23-12-1999 | CA | 2335782 A1 | 23-12-1999 |
| | | EP | 1087697 A1 | 04-04-2001 |
| | | JP | 2002518077 A | 25-06-2002 |
| | | US | 6537228 B1 | 25-03-2003 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**

EP 15 16 2130

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-08-2015

| Im Recherchenbericht angeführtes Patentdokument | Datum der Veröffentlichung | Mitglied(er) der Patentfamilie | Datum der Veröffentlichung |
|---|---|---|---|
| | | WO 9965393 A1 | 23-12-1999 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10301202